# EUROPEAN PATENT APPLICATION

(11) **EP 2 535 455 A1**
(43) Date of publication of application: **19.12.2012**
(21) Application number: 11170028.2
(22) Date of filing: 15.06.2011
(51) Int. Cl.: D21H 21/30, C07D 251/00, C09K 11/06

(54) **Use of fluorescent whitening agent compositions for whitening paper**

(71) Applicant: Blankophor GmbH & Co. KG, 49577 Ankum (DE)
(72) Inventor: Hunke, Bernhard, 53773 Hennef (DE); Tauber, Andrei, 50735 Köln (DE); Kraemer, Michael, 51515 Kürten (DE); Klug, Günter, 40764 Langenfeld (DE)
(74) Representative: Bublak, Wolfgang

(57) **Abstract**

The invention relates to the use of a composition for optically whitening paper or board, wherein the composition comprises specific sulfo-type bis-triazinylamino-stilbene compounds.

## Description

The present invention relates to the use of fluorescent whitening agent compositions comprising specific sulfo-type bis-triazinylamino-stilbene compounds for whitening paper or board.

It is well known that the whiteness of paper and board can be improved by the addition of fluorescent whitening agents (FWAs). The most important fluorescent whitening agents used in the paper and board industry are anilino-substituted bistriazinyl derivatives of 4,4'-diaminostilbene-2,2'-disulfonic acid (flavonic acid). From these fluorescent whitening agents disulfo-, tetrasulfo- and hexasulfo-types are known. The disulfo-type fluorescent whitening agents with no sulfonic acid groups at the aniline rings have a low solubility in water and a high affinity for cellulose fibres. They are especially suitable for use at the wet-end of paper making process. The hexasulfo-type fluorescent whitening agents with two sulfonic acid groups at each aniline ring have a high solubility in water and a low affinity for cellulose fibres. They are more specialty products when very high whiteness is desired. The tetrasulfo-type fluorescent whitening agents with one sulfonic acid group at each aniline ring exhibit a behaviour between the disulfo- and hexasulfo-type fluorescent whitening agents and are most commonly used for whitening paper or board.

Document DE 1 811 715 A1 refers to specific bis-triazinylamino-stilbene compounds of the tetrasulfo-type, in particular mixtures thereof, wherein the sulfonic acid group at the respective terminal phenyl ring is in *para-*or *meta*-position and which are produced by using a mixture of *para-*aminobenzene sulfonic acid and *meta*-aminobenzene sulfonic acid in specified ratios. The aqueous solutions of the disclosed mixtures are described to be stable at acidic pH values and are used for the simultaneous optical brightening and high-quality finishing of textile materials.

In the paper-producing industry there is a continuing trend towards papers having high whiteness. Whitening paper is usually carried out by using a fluorescent whitening agent (FWA) either before the sheet formation in the paper pulp or after sheet formation, in the size press or by coating compositions. A combination of more than one of said processes is also possible.

Surprisingly, it has been found that bis-triazinylamino-stilbene compounds containing sulfonic acid groups at specific positions of the terminal phenyl rings when used for whitening paper or board yield paper or board of improved whiteness.

Therefore, the present invention relates to the use of a fluorescent whitening agent composition for optically whitening paper or board, wherein the composition contains at least two fluorescent whitening agents selected from the fluorescent whitening agents of the formula (1), the formula (2), and the formula (3) 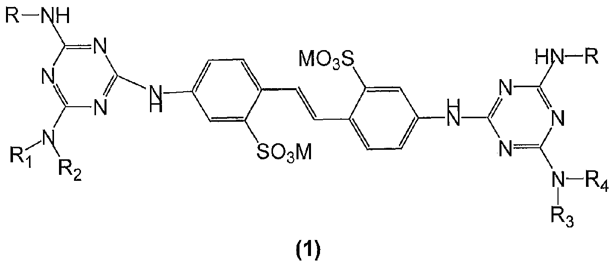 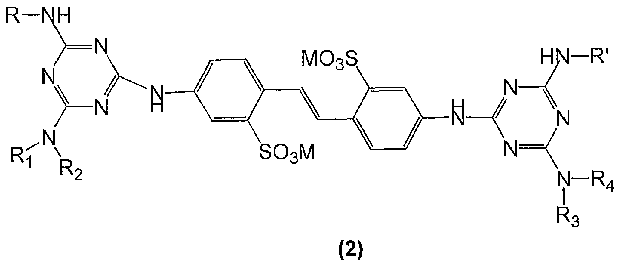 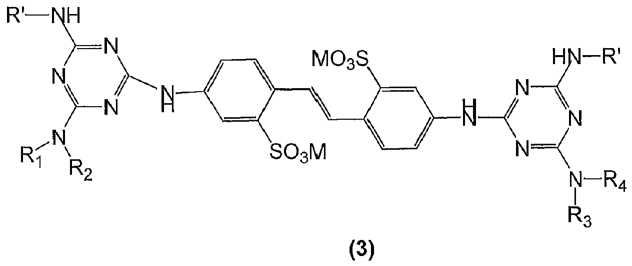 Wherein
R stands for the radical 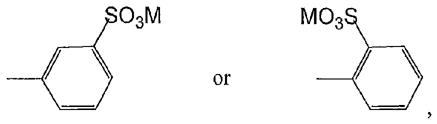 and R' stands for the radical 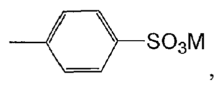 or R stands for the radical 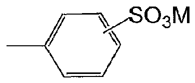 and R' stands for the radical 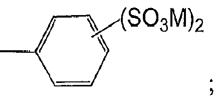 wherein
R₁, R₂, R₃ and R₄ represent, independently of each other, hydrogen, cyano, C₁-C₄ alkyl, C₂-C₄ hydroxyalkyl, C₂-C₄ cyanoalkyl or C₁-C₄ alkoxyalkyl, or the residue of an amino acid from which a hydrogen atom has been removed from the amino group; or R₁ and R₂, or R₃ and R₄, independently of each other, together with N atom form morpholine, piperidine or pyrrolidine ring; or -(CH₂)₁-SO₃M, where 1 is 1, 2 or 3; or -(CH₂)ᵢ-COOR, -(CH₂)ᵢ-CONHR, -(CH₂)ᵢ-COR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁-C₄ alkyl or C₂-C₄ hydroxyalkyl.

The invention further relates to a process for optically whitening paper or board, wherein a pulp and/or a cellulose sheet is brought into contact with the above defined composition. Further, the invention refers to paper obtainable by this process. Preferred embodiments of the invention are described in the description (including the examples) hereinafter, the claims and the figure.

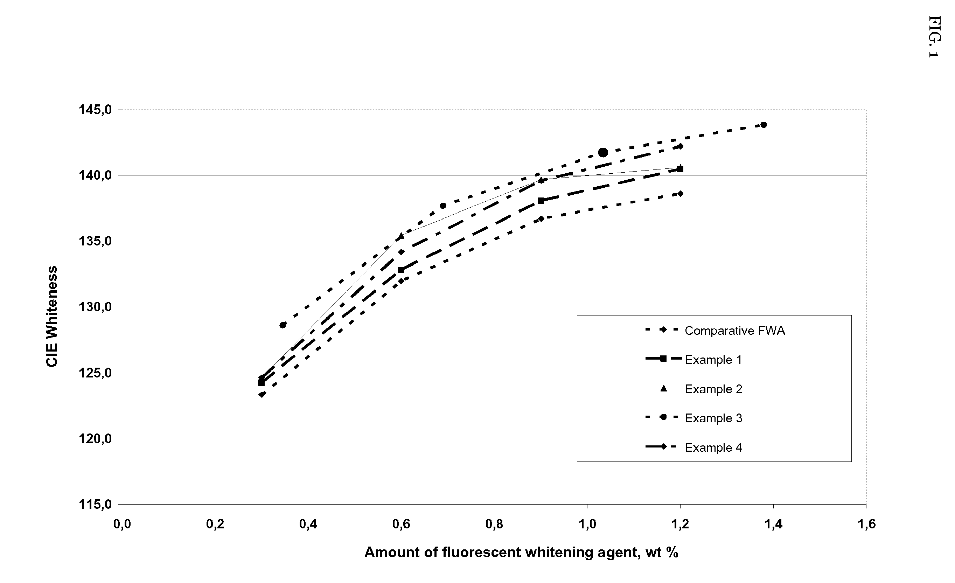

Fig. 1 is a diagram showing the whitening performance of different fluorescent whitening agents.

In a preferred embodiment of the invention, the used composition is a wet-end composition, and the process is a process for whitening paper, wherein a pulp or pulp suspension is brought into contact with said composition.

In another preferred embodiment of the invention, the used composition is a size press liquor, and the process is a process for whitening paper in the size press, wherein a cellulose sheet is brought into contact with the size press liquor.

In the context of this invention, size press is understood as meaning a surface application unit, preferably of the paper machine, in which the cellulose sheet formed is brought into contact with a size press liquor, and in which the proportion of the liquor which is to be taken up by the sheet (liquor absorption) can preferably be adjusted by means of the roll pressure. Recent developments of the size press or film press, namely of the Speedsizer as well as of the Symsizer as well Gate-roll, are likewise understood as being covered by the term size press.

In another preferred embodiment of the invention, the used composition is a coating composition or coating slip, and the process is a process for whitening paper, wherein a cellulose sheet is brought into contact with the coating composition or coating slip.

According to the invention the composition or mixture used contains at least two of the bis-triazinylamino-stilbene compounds of the above defined formulae (1), (2) and (3). In the context of the invention, in the formulae (1), (2) and (3) the alkyl group can be linear or branched, and the possible substituents of the alkyl group, which are alkoxy, cyano, and/or hydroxyl groups, can be attached at any position of the alkyl chain. In the present invention, C₁-C₄ alkoxyalkyl means C₁-C₄ alkyl substituted with C₁-C₄ alkoxy. In a preferred embodiment, R₁, R₂, R₃ and R₄ represent, independently of each other, C₂-C₄ hydroxyalkyl, C₁-C₄ alkoxyalkyl, or C₁-C₄ alkyl, preferably C₂-C₄ hydroxyalkyl or C₁-C₄ alkoxyalkyl, in particular hydroxyethyl or hydroxyisopropyl. Most preferably, R₁, R₂, R₃ and R₄ represent hydroxyethyl.

In a preferred embodiment, R' has one -SO₃M group. In another preferred embodiment, R' has two -SO₃M groups. In case that R' has two -SO₃M groups those groups can be at any position of the terminal phenyl rings, and in this case also for R the single -SO₃M group can be at any position of the terminal phenyl rings. Preferred compounds are those where for R the -SO₃M group is in *para-* or *meta*-position, and for R' the two -SO₃M groups are, independently of each other, in *ortho-* and *para-*positions or in *meta-* and *meta*-positions. Preferably, for R the -SO₃M group is in *para*-position and for R' the two -SO₃M groups are in *meta-*and *ortho*-positions.

Preferred embodiments of M are hydrogen, Na, K, Ca, Mg, in particular M is Na, K or hydrogen, most preferred is Na. The fluorescent whitening agents are used as free acids or as salts thereof, preferably alkali metal salts.

The fluorescent whitening agents of formulae (1), (2) and (3) and the mixtures thereof can be prepared by known procedures. Generally, the compounds are prepared by reacting cyanuric chloride with 4,4'-diaminostilbene-2,2'-disulfonic acid or a salt thereof, aminobenzene sulfonic acid and/or the corresponding aniline compound with two sulfonic acid groups, and substituted aliphatic amines or heterocyclic compounds. The ratio of aminobenzene sulfonic acid and/or aniline compound with two sulfonic acid groups can be selected such that a desired ratio of the fluorescent whitening agents of formulae (1), (2) and/or (3) is obtained.

The composition of the fluorescent whitening agents of formulae (1), (2) and/or (3) can be produced in form of a mixture with the desired ratio of the fluorescent whitening agents of formulae (1), (2) and/or (3). Alternatively, the composition can be produced by preparing separately the fluorescent whitening agents of formulae (1), (2) and/or (3) by the methods known in the art and as described herein, and then blending or mixing together in the desired ratio after their preparation. Suitable processes are also described in DE 1 811 715 A1.

The fluorescent whitening agent composition contains at least two, in particular at least three fluorescent whitening agents selected from the fluorescent whitening agents of formulae (1), (2) and (3). In a preferred embodiment, the composition contains two fluorescent whitening agents selected from the fluorescent whitening agents of formulae (1), (2) and (3). In another preferred embodiment, the composition contains three fluorescent whitening agents selected from the fluorescent whitening agents of formulae (1), (2) and (3). In a more preferred embodiment, the composition comprises the fluorescent whitening agents of the formulae (1) and (2). In a further more preferred embodiment, the composition comprises the fluorescent whitening agents of the formulae (2) and (3). In another more preferred embodiment, the composition comprises the fluorescent whitening agents of the formulae (1) and (3). In a most preferred embodiment, one of the at least two fluorescent whitening agents of the formulae (1), (2) and/or (3) is the compound of formula (2). In another most preferred embodiment, the composition comprises the fluorescent whitening agents of the formulae (1), (2) and (3). The composition can also contain one or more of each of a fluorescent whitening agent of the formulae (1), (2) and/or (3). In addition, the composition can contain one or more known bis-triazinylamino-stilbene or distyryl-biphenyl based fluorescent whitening agents.

Preferred compositions used according to the invention are shown below in the Embodiments 1 and 2, wherein R₁, R₂, R₃, R₄ and M are as described above.

### Embodiment 1

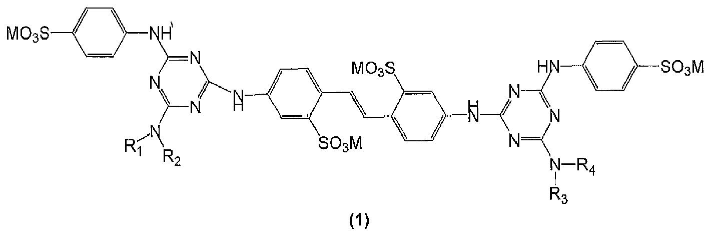

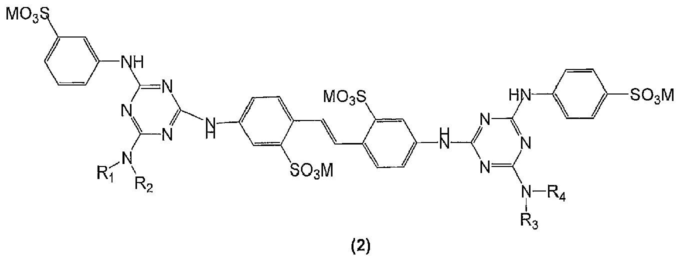

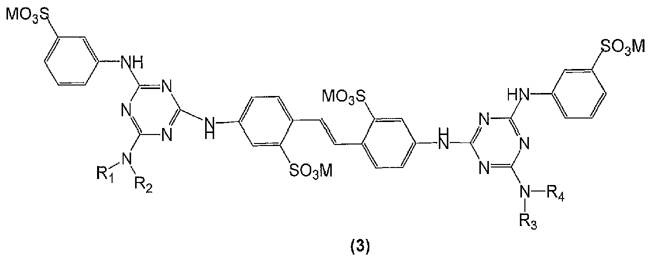

### Embodiment 2

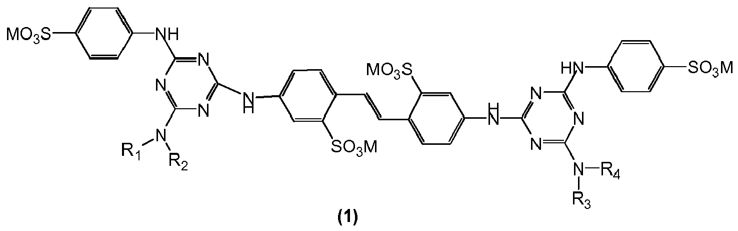

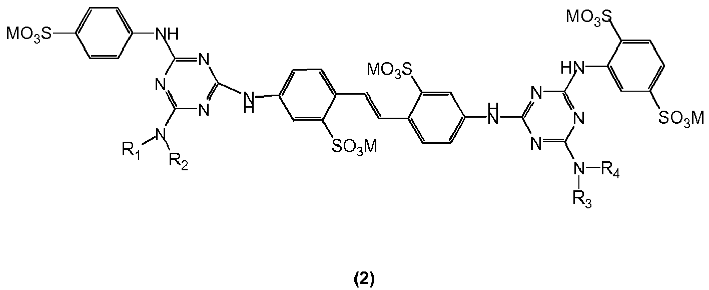

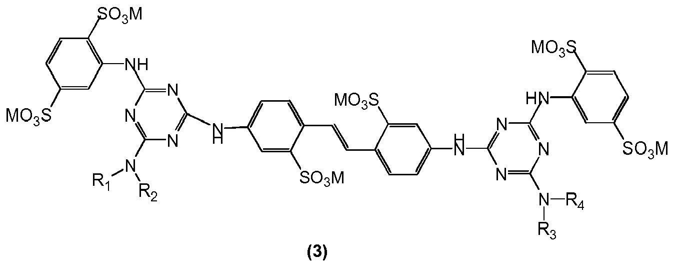

The amounts of the fluorescent whitening agents present in the composition depend on the number of fluorescent whitening agents present and which fluorescent whitening agents are present. According to the invention the composition used contains preferably at least one fluorescent whitening agent of formula (1) in an amount of 0 to 99 weight-%, preferably 2 to 90 weight-%, most preferably 5 to 80 weight-%; at least one fluorescent whitening agent of formula (2) in an amount of 0 to 99 weight-%, preferably 2 to 90 weight-%, most preferably 5 to 80 weight-%; and at least one fluorescent whitening agent of formula (3) in an amount of 0 to 99 weight-%, preferably 2 to 90 weight-%, most preferably 5 to 80 weight-%; in each case based on 100 weight-% of the total amount of the present fluorescent whitening agents of the formulae (1), (2) and/or (3). In further preferred embodiments the composition contains preferably at least one fluorescent whitening agent of formula (1) in an amount of 10 to 80 weight-%, in particular 20 to 70 weight-%, at least one fluorescent whitening agent of formula (2) in an amount of 10 to 60 weight-%, in particular 10 to 50 weight-%, and at least one fluorescent whitening agent of formula (3) in an amount of 10 to 80 weight-%, in particular 20 to 70 weight-%, in each case based on 100 weight-% of the total amount of the present fluorescent whitening agents of the formulae (1), (2) and/or (3).

The fluorescent whitening agent composition may be used in any commercially available form, e.g. as powders or granules, which may be dissolved in water, or it may be used in the form of an aqueous preparation, an aqueous solution, or an aqueous dispersion, or an aqueous preparation directly from the production. In a preferred embodiment, the composition is an aqueous preparation. In a further preferred embodiment, the composition contains water, preferably in an amount of 40 to 99 weight-%, preferably 60 to 97 weight-%, most preferably 70 to 95 weight-%, in each case based on 100 weight-% composition.

Depending on its use, the fluorescent whitening agent composition can contain carriers, sizing agents, a salt of a bivalent cation, and in addition, in relatively small amounts, usually in amounts of less than 10% by weight. Further auxiliaries, such as, for example, dispersants, thickeners, antifreezes, preservatives, complexing agents, etc., or organic byproducts from the fluorescent whitening agent synthesis which were not completely removed in the working-up, may be contained in the composition. In a preferred embodiment of the invention, the composition is a size press liquor containing the fluorescent whitening agents as defined above, water, and at least one carrier and/or sizing agent. In another preferred embodiment of the invention, the composition is a coating composition or coating slip containing the fluorescent whitening agents as defined above, water, at least one binder and/or pigment and, optionally, at least one carrier and/or co-binder.

Suitable carriers are any compounds known in the art to be suitable as carrier, in particular carriers suitable for size press liquors or coating compositions. Preferred carriers are carboxymethylcellulose (CMC), polyvinyl alcohol (PVA), starch or mixtures thereof, with starch being particularly preferred. Suitable carrier substances are, for example, hydrophilic polymers having the ability to form hydrogen bridge bonds. Preferred carrier substances are starch, polyvinyl alcohols, carboxymethylcelluloses and polyethylene glycols having a number average molecular weight of from 200 to 8000 g/mol, as well as any desired mixtures of these substances, it being possible for these polymers optionally to be modified. Preferred polyvinyl alcohols are those having a degree of hydrolysis >85%, preferred carboxymethylcelluloses are those having a degree of substitution DS of >0.5. Polyethylene glycols having a number average molecular weight Mn of from 200 to 8000 g/mol are particularly preferred. Suitable starches are based e.g., but not exclusively, on potato starch, rice starch, wheat starch, maize starch or tapioca starch. In particular, starches whose molecular weights have already been reduced by partial degradation and/or which have been obtained by derivatization are preferably used instead of natural starches. Furthermore, starches for which both modification steps have been combined, i.e. which have been partially degraded and additionally derivatized, are suitable. Typical methods for starch degradation are, for example, enzymatic, oxidative, thermal or hydrolytic treatment. Examples of suitable starch derivatives are hydroxyethyl starch or cationic starch.

Suitable sizing agents are alkenyl ketene dimers, alkyl ketene dimer (AKD), alkenyl succinic anhydride (ASA), rosin size, styrene maleic anhydride copolymers, styrene acrylate, styrene acrylic acid copolymers, polyurethane or ethylene acrylic acid copolymers, or other common paper chemicals, such as styryl-acrylate copolymers, latex, pigments, defoamers, or salts, such as NaCl or NaHCO₃, or mixtures of two or more thereof. If sizing agents are used, they are used in amounts of 0 to 5, in particular 0 to 4, most preferably 0 to 3, in each case weight-% based on 100 weight-% of the composition.

The coating slips or coating compositions contain, as latex binder, for example lattices based on styrene/butadiene, styrene/acrylate or vinyl acetate. These polymers can optionally be modified by further monomers, such as acrylonitrile, acrylamide, α,β-unsaturated carboxylic acids, such as acrylic acid, methacrylic acid, itaconic acid or maleic acid, acrylates, vinyl esters, ethylene, vinyl chloride, vinylidene chloride, etc. In general, however, all customary latex binders which are used for the preparation of paper coating slips or coating compositions are suitable. The coating slips or coating compositions can contain as synthetic co-binders differing from these, for example, carboxymethylcellulose, hydroxylalkylcellulose and/or polyvinyl alcohol and acrylate-based synthetic thickeners.

Preferred latex binders are those based on styrene/butadiene. Preferred synthetic co-binders are polyvinyl alcohols, in particular those having a degree of hydrolysis of >85%, and in particular a Brookfield viscosity of 2-80 mPas (measured on a 4% strength aqueous solution at 20° C), carboxymethylcelluloses, in particular those having a degree of substitution of >0.5, and in particular a Brookfield viscosity of from about 5 to about 5000 mPas (measured on a 2% strength aqueous solution at 20° C) and mixtures of these two substances.

The coating slips or coating compositions preferably furthermore contain white pigments. Customarily used white pigments are calcium carbonate in natural or precipitated form, kaolin, talc, titanium dioxide, satin white, aluminum hydroxide, zinc sulfide, calcium sulfate, and barium sulfate, often also in the form of mixtures thereof.

The production of the composition used according to the invention may be effected by known methods and preferably effected by combining an aqueous solution of the fluorescent whitening agents used, which solution preferably has a suitable pH value, with the other components, such as carrier substances, sizing agents, binders, pigments, salts or standardizing agents.

The process for whitening paper is carried out according to known processes in wet-end and/or surface applications, whereas surface application could be coating or by using a size press, and is subject to no restrictions. The pulp or paper used is not critical and may be any pulp, pulp furnish, pulp suspension, or cellulose sheet.

Paper obtained by the whitening process of the invention exhibits improved whiteness.

The whiteness of the papers produced can be characterized by the CIE whiteness. Different fluorescent whitening agents can be compared to each other with respect to the saturation behavior when determined according to CIE whiteness. In other words, if a larger amount of fluorescent whitening agent is used and no further increase in whiteness is found, there is a saturation behavior and there may even be adverse effects on the whiteness when using higher amounts. The effect of saturation is also referred to as greening. The greening limit, i.e. the point at which increasing amounts of fluorescent whitening agent used results in virtually no further increase in whiteness, can be derived, for example, from the a*-b* diagram, where a* and b* are the color coordinates in the CIE-L*a*b* system.

The following examples illustrate the invention and show preferred embodiments, without limiting the scope of protection.

### EXAMPLES

### Example 1 (R₁, R₂, R₃, R₄ = -CH₂CH₂OH)

### Step 1: Reaction of cyanuric chloride with 2,2'-disulfo-4,4'-stilbenediamine disodium salt

A two-liter flask equipped with an agitator, pH electrode, thermometer and condenser was charged with 600 ml of water at 8 °C, and 100 g (0.542 mol) of cyanuric chloride. After the pH decreased to 4.5, a solution of 112.2 g (0.27 mol) of 2,2'-disulfo-4,4'-stilbenediamine disodium salt in 835 g water was added dropwise. At the same time 10 % (w/w) solution of sodium hydroxide (222 g) was added dropwise to the reaction mixture to keep the pH at 4.5 while heating the mixture up to 16 °C. After addition of the reagents the reaction mixture was stirred at 16 °C for 1 hour while maintaining pH 4.5.

### Step 2: Reaction of Step 1 intermediate with 3-aminobenzenesulfonic acid

627.9 g of about 15 % (w/w) solution of 3-aminobenzenesulfonic acid (0.54 mol) were added to the water suspension from Step 1 in 30 min. The pH was kept constant (pH = 4.5) by the simultaneous addition of 10 % sodium hydroxide solution while temperature was gradually increased up to 65 °C. The reaction mixture was then stirred at 65 °C and pH 4.5 until the consumption of the sodium hydroxide solution has stopped.

### Step 3: Reaction of the Step 2 intermediate with diethanolamine

68.1 g (0.65 mol) of diethanolamine were added to the water suspension from Step 2 in 20 minutes. The pH was kept constant (pH = 8) by the simultaneous addition of 10 % sodium hydroxide solution while temperature was gradually increased up to 100 °C. The reaction mixture was then stirred at 100 °C and pH 8 for 3 hours. The resulting solution was cooled down to about 50 °C and clarified by filtration to yield the solution of compound of the formula 1 (92.5%).

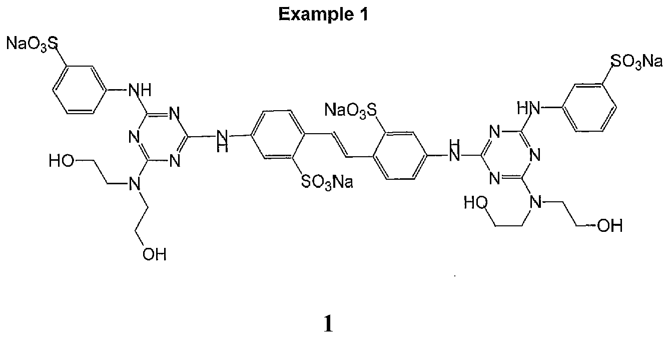

### Example 2 (R₁, R₂, R₃, R₄ = -CH₂CH₂OH)

### Step 1: Conditions are identical with those from Step 1 of Example 1

### Step 2: Reaction of Step 1 intermediate with mixture of 3-aminobenzenesulfonic and 4-aminobenzenesulfonic acids (3/2, w/w)

623.3 g (0.54 mmol) of a mixture of 3-aminobenzenesulfonic acid and 4-aminobenzenesulfonic acid (3/2, w/w) were added as about 15 % (w/w) solution to the water suspension from Step 1 in 45 min. The pH was kept constant (pH = 4.5) by the simultaneous addition of 10 % sodium hydroxide solution while temperature was gradually increased up to 60 °C. The reaction mixture was then stirred at 60 °C and pH 4.5 until the consumption of the sodium hydroxide solution has stopped.

### Step 3: Reaction of the Step 2 intermediate with diethanolamine

68.1 g (0.65 mol) of diethanolamine were added to the water suspension from Step 2 in 20 minutes. The pH was kept constant (pH = 8) by the simultaneous addition of 10 % sodium hydroxide solution while temperature was gradually increased up to 100 °C. The reaction mixture was then stirred at 100 °C and pH 8 for 3 hours. The resulting solution was cooled down to about 50 °C and clarified by filtration to yield a solution of the following mixture with the compounds of formula **1** (20%), **2** (45.2%) and **3** (34.8%) in an overall yield of 86.3%.

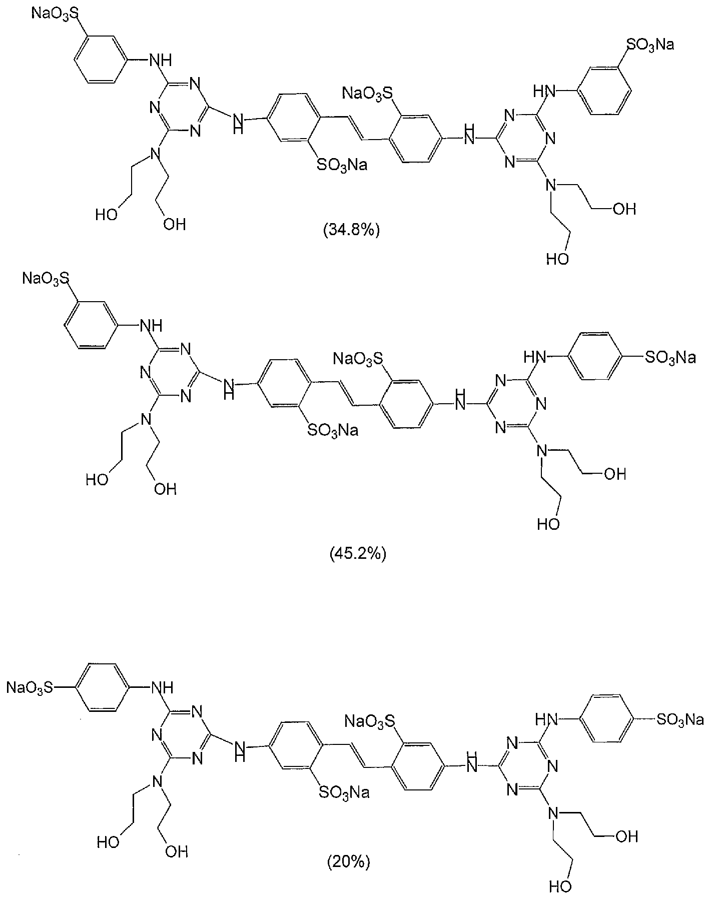

### Example 3 (R₁, R₂, R₃, R₄ = -CH₂CH₂OH)

### Step 1: Conditions are identical with those from Step 1 of Example 1

### Step 2: Reaction of Step 1 intermediate with mixture of 3-aminobenzenesulfonic and 4-aminobenzenesulfonic acids (7/3, w/w)

626.5 g (0.54 mmol) of a mixture of 3-aminobenzenesulfonic acid and 4-aminobenzenesulfonic acid (7/3, w/w) were added as about 15 % (w/w) solution to the water suspension from Step 1 in 45 min. The pH was kept constant (pH = 4.5) by the simultaneous addition of 10 % sodium hydroxide solution while temperature was gradually increased up to 60 °C. The reaction mixture was then stirred at 60 °C and pH 4.5 until the consumption of the sodium hydroxide solution has stopped.

### Step 3: Reaction of the Step 2 intermediate with diethanolamine

68.1 g (0.65 mol) of diethanolamine were added to the water suspension from Step 2 in 20 minutes. The pH was kept constant (pH = 8) by the simultaneous addition of 10 % sodium hydroxide solution while temperature was gradually increased up to 100 °C. The reaction mixture was then stirred at 100 °C and pH 8 for 3 hours. The resulting solution was cooled down to about 50 °C and clarified by filtration to yield a solution of the following mixture with the compounds of formula **1** (12%), **2** (38.5%) and **3** (49.5%) in an overall yield of 91.7%.

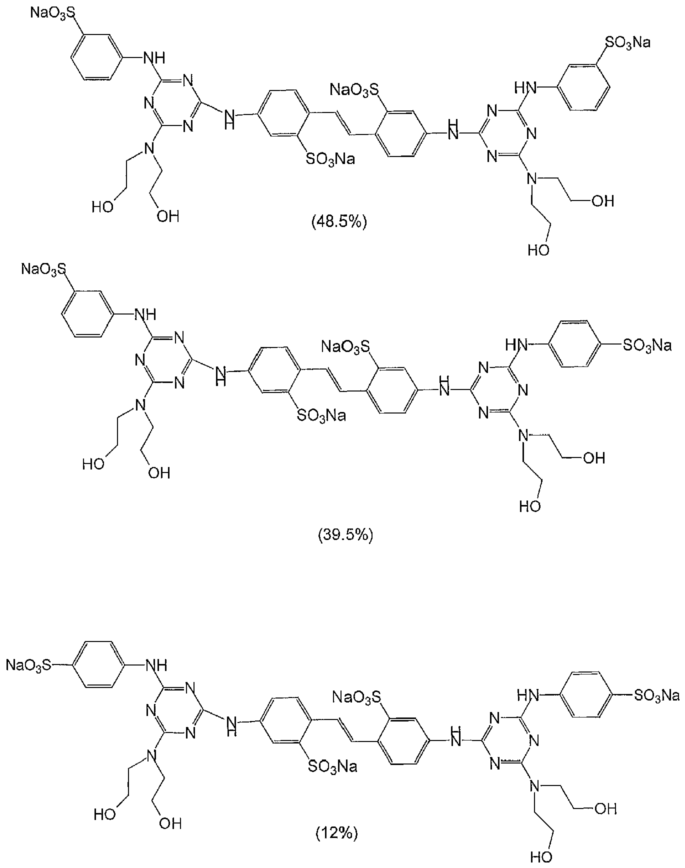

### Example 4 (R₁, R₂, R₃, R₄ = -CH₂CH₂OH)

### Step 1: Conditions are identical with those from Step 1 of Example 1

### Step 2: Reaction of Step I intermediate with mixture of 3-aminobenzenesulfonic and 4-aminobenzenesulfonic acids (1/1, w/w)

619.7 g (0.54 mmol) of a mixture of 3-aminobenzenesulfonic acid and 4-aminobenzenesulfonic acid (1/1, w/w) were added as about 15 % (w/w) solution to the water suspension from Step 1 in 45 min. The pH was kept constant (pH = 4.5) by the simultaneous addition of 10 % sodium hydroxide solution while temperature was gradually increased up to 60 °C. The reaction mixture was then stirred at 60 °C and pH 4.5 until the consumption of the sodium hydroxide solution has stopped.

### Step 3: Reaction of the Step 2 intermediate with diethanolamine

68.1 g (0.65 mol) of diethanolamine were added to the water suspension from Step 2 in 20 minutes. The pH was kept constant (pH = 8) by the simultaneous addition of 10 % sodium hydroxide solution while temperature was gradually increased up to 100 °C. The reaction mixture was then stirred at 100 °C and pH 8 for 3 hours. The resulting solution was cooled down to about 50 °C and clarified by filtration to yield a solution of the following mixture with the compounds of formula **1** (30.1%), **2** (44.7%) and **3** (25.2%) in an overall yield of 89.5%.

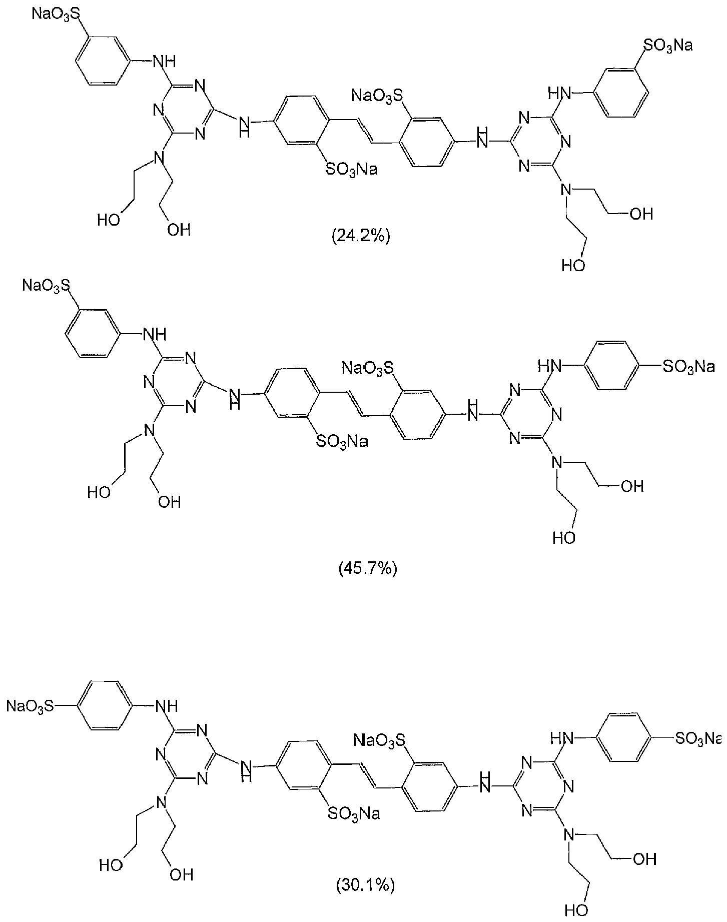

These fluorescent whitening agents and mixtures were used in the following application examples. The following fluorescent whitening agent was used as Comparative FWA:

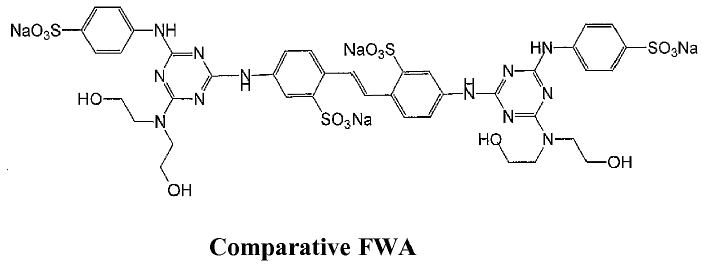

### Application Example

The whitening performance of the fluorescent whitening agents and mixtures thereof was studied using the following test procedure.

The furnish (pulp suspension) used was a composition of bleached pulp and composed of 70 parts of hardwood and 30 parts of softwood with a grinding degree of 35 °SR (Schopper-Riegler). 800 ml of 0.625% of that furnish were weighted in a beaker to prepare a 5 g hand sheet of about 120 g/m² for each experimental series. Solutions with 0.1 weight (wt) % of the respective fluorescent whitening agent (FWA) were prepared using distilled water. The amounts of FWA as indicated in Table 1 below were achieved by adding a corresponding amount from the respective 0.1 wt % fluorescent whitening agent solution by a pipette to the stirred pulp suspension which was stirred for 10 minutes after fluorescent whitening agent addition. The amounts of fluorescent whitening agents in Table 1 are calculated as active ingredient based on 100 weight % of dry pulp.

A wet filter paper was positioned on the wire of the sheet former, the stock was put on the sheet former and sucked dry. The formed hand sheet was protected by an additional dry filter, pressed and dried on a calender at 100°C. Thereafter, the obtained hand sheets were equilibrated in a climate room under standard conditions overnight and then measured with a Datacolor spectrometer (ISO2469) by determining CIE, L*, a* and b*, the light source used being based on ISO2469 standard.

The results obtained are summarized in Table 1 and further shown in Fig. 1.

**TABLE 1**

| FWA | Amount (wt %) of FWA | CIE whiteness | L* | a* | b* |
|---|---|---|---|---|---|
| Example 1 | 0.3 | 124.26 | 97.35 | 2.05 | -7.02 |
| | 0.6 | 132.82 | 97.68 | 2.49 | -8.81 |
| | 0.9 | 138.05 | 97.82 | 2.69 | -9.94 |
| | 1.2 | 140.48 | 97.97 | 2.73 | -10.43 |
| Example 2 | 0.3 | 124.60 | 97.57 | 2.08 | -6.98 |
| | 0.6 | 135.44 | 97.89 | 2.56 | -9.31 |
| | 0.9 | 139.65 | 97.92 | 2.61 | -10.27 |
| | 1.2 | 140.60 | 98.05 | 2.52 | -10.42 |
| Example 3 | 0.35 | 128.60 | 97.63 | 2.35 | -7.87 |
| | 0.69 | 137.65 | 97.86 | 2.70 | -9.83 |
| | 1.03 | 141.71 | 97.98 | 2.76 | -10.71 |
| | 1.38 | 143.82 | 98.21 | 2.61 | -11.08 |
| Example 4 | 0.4 | 124.64 | 97.56 | 2.12 | -6.99 |
| | 0.6 | 134.16 | 97.82 | 2.54 | -9.05 |
| | 0.9 | 139.58 | 97.96 | 2.70 | -10.23 |
| | 1.2 | 142.18 | 98.06 | 2.65 | -10.78 |
| Comparative FWA | 0.3 | 123.32 | 97.28 | 2.02 | -6.84 |
| | 0.6 | 131.95 | 97.70 | 2.45 | -8.60 |
| | 0.9 | 136.69 | 97.83 | 2.59 | -9.63 |
| | 1.2 | 138.58 | 97.90 | 2.58 | -10.03 |

Thus, the fluorescent whitening agent compositions or mixtures of Examples 2, 3 and 4 (invention) exhibit better whitening performance than the Comparative FWA or the FWA of Example 1.

## Claims

1. Use of a fluorescent whitening agent composition for optically whitening paper or board, wherein the composition contains at least two fluorescent whitening agents selected from the fluorescent whitening agents of the formula (1), the formula (2), and the formula (3) 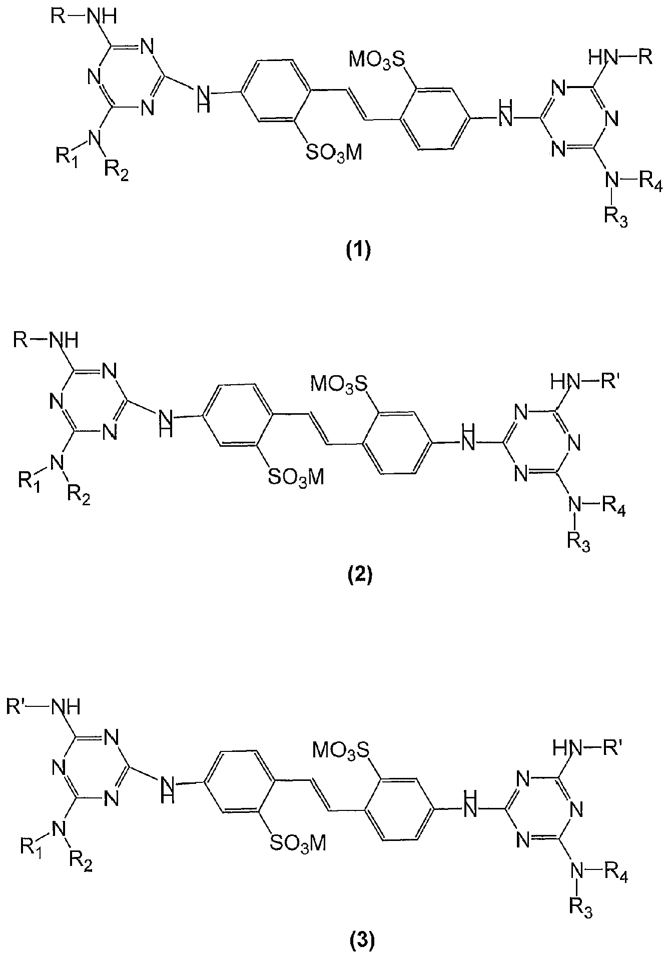 Wherein
R stands for the radical 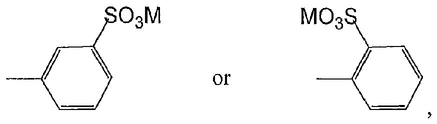 and R' stands for the radical 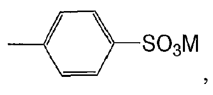 or R stands for the radical 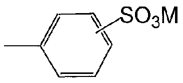 and R' stands for the radical 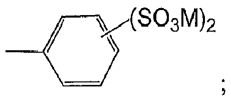 wherein
R₁, R₂, R₃ and R₄ represent, independently of each other, hydrogen, cyano, C₁-C₄ alkyl, C₂-C₄ hydroxyalkyl, C₂-C₄ cyanoalkyl or C₁-C₄ alkoxyalkyl, or the residue of an amino acid from which a hydrogen atom has been removed from the amino group; or R₁ and R₂, or R₃ and R₄, independently of each other, together with N atom form morpholine, piperidine or pyrrolidine ring; or -(CH₂)₁-SO₃M, where 1 is 1, 2 or 3; or -(CH₂)ᵢ-COOR, -(CH₂)ᵢ-CONHR, -(CH₂)ᵢ-COR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁-C₄ alkyl or C₂-C₄ hydroxyalkyl.

2. The use according to claim 1, wherein R₁, R₂, R₃ and R₄ represent, independently of each other, C₂-C₄ hydroxyalkyl, or C₁-C₄ alkoxyalkyl.

3. The use according to claim 1 or 2, wherein R' has one -SO₃M group.

4. The use according to claim 1 or 2, wherein R' has two -SO₃M groups.

5. The use according to any of the preceding claims, wherein one of the at least two fluorescent whitening agents is the fluorescent whitening agent of formula (2).

6. The use according to any of the preceding claims, wherein the composition contains the fluorescent whitening agents of formula (1), formula (2) and formula (3).

7. The use according to any of the preceding claims, wherein the composition contains the following fluorescent whitening agents:

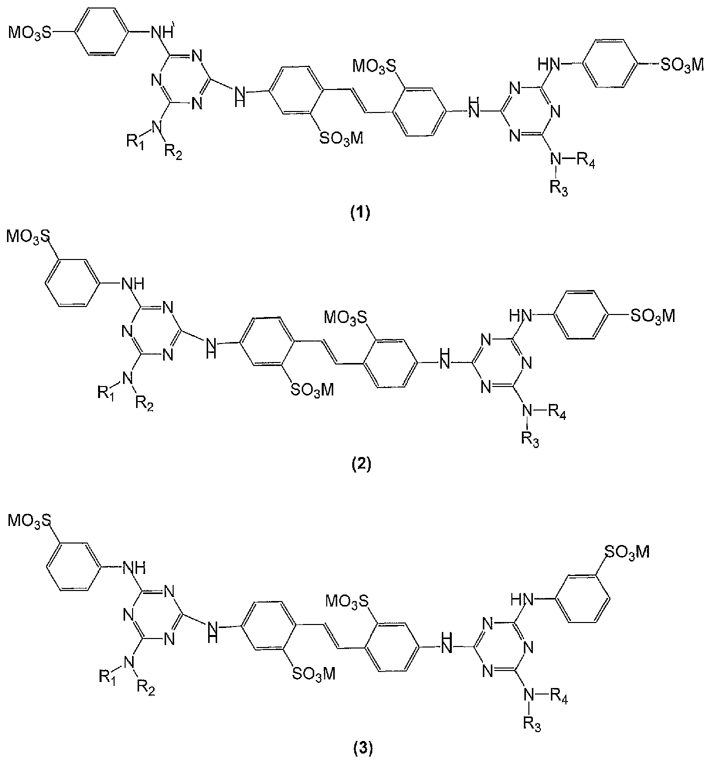

8. The use according to any of claims 1 to 5, wherein the composition contains the following fluorescent whitening agents:

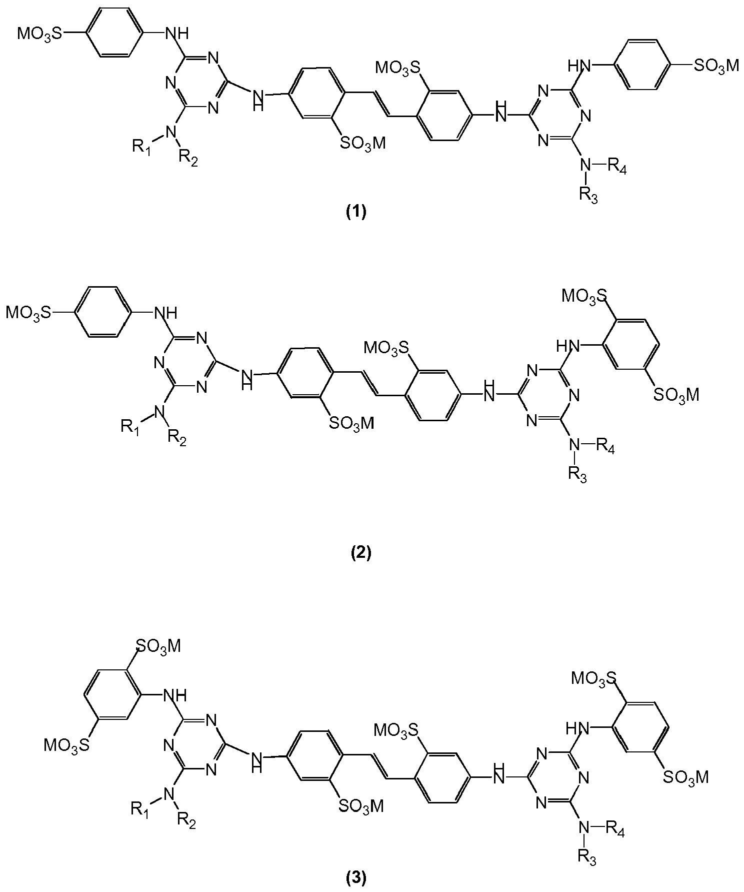

9. The use according to any of the preceding claims, wherein the composition contains the fluorescent whitening agent of formula (1) in an amount of 2 to 90 weight-%, the fluorescent whitening agent of formula (2) in an amount of 2 to 90 weight-%, and the fluorescent whitening agent of formula (3) in an amount of 2 to 90 weight-%, in each case based on 100 weight-% of the total amount of the present fluorescent whitening agents of the formula (1), (2) and/or (3).

10. A process for preparing a fluorescent whitening agent composition as defined in any of the preceding claims, by reacting cyanuric chloride with 4,4'-diaminostilbene-2,2'-disulfonic acid or a salt thereof, aminobenzene sulfonic acid and/or the corresponding aniline compound with two sulfonic acid groups, and substituted aliphatic amines or heterocyclic compounds.

11. A process for whitening paper or board, wherein a pulp or pulp suspension is brought into contact with a composition as defined in any of claims 1 to 9.

12. A process for whitening paper or board, wherein a cellulose sheet is brought into contact with a composition as defined in any of claims 1 to 9.

13. A paper obtainable by the process according to any of claims 10 to 12.
